# EUROPEAN PATENT SPECIFICATION

(11) **EP 2 967 583 B1**
(45) Date of publication and mention of the grant of the patent: **09.05.2018**
(21) Application number: 14711074.6
(22) Date of filing: 28.02.2014
(51) Int. Cl.: A61B 17/16

(54) **BONE CUTTING DEVICE**
KNOCHENSCHNEIDEVORRICHTUNG
DISPOSITIF DE COUPE D'OS

(30) Priority: 13.03.2013 US 201361778931 P
(43) Date of publication of application: 20.01.2016
(73) Proprietor: DePuy Synthes Products, Inc., Raynham, MA 02767 (US)
(72) Inventor: KOAY, Kenny, West Chester, PA 19380 (US); MCMILLAN, Rod, West Chester, PA 19380 (US); HAAG, Rene, West Chester, PA 19380 (US)
(74) Representative: Klunker IP Patentanwälte PartG mbB
(86) International application number: PCT/US2014/019261
(87) International publication number: WO 2014/158683

(56) References cited:
- EP-A1- 2 543 326
- WO-A2-02/09598
- WO-A2-2007/136777
- US-A- 5 152 774
- US-A- 6 033 408

## Description

### Background

Bone cutting devices such as drills, reamers, chisels and needles are typically formed of metal to permit the devices to withstand forces applied thereto as they are applied in bone fixation procedures. Generally, such bone cutting devices are not constructed out of implant-grade metal since implant-grade metal is not strong enough to withstand the forces repeatedly applied during use. Thus, the use of drills and reamers constructed out of implant-grade metal would result in damage to the device and require frequent replacement.

Typically, tools used in bone fixation such as drill and reamers are constructed of non-implant-grade metal. However, if such bone cutting devices fracture of break during surgical use, fragments may be dislodged into the body. The time required to locate and remove such non-implant-grade metal fragments is lengthy and may exceed the time required to perform a target bone fixation procedure.
EP 2 543 326 A1 discloses an orthopaedic drill bit that may be formed of austenitic stainless steel having a treated surface to improve corrosion resistance. The features of the preamble of claim 1 are known from WO 2007/136777 A2.

### Summary of the Invention

The present invention is directed to a bone cutting device according to the features of claim 1.

### Brief Description of the Drawings

Fig. 1 shows a side view of a reamer head according to an exemplary embodiment of the present invention;
Fig. 2 shows a perspective view of the reamer head of Fig. 1;
Fig. 3 shows a side view of a reamer head according to a second exemplary embodiment of the invention;
Fig. 4 shows a side view of a reamer head according to a third exemplary embodiment of the invention;
Fig. 5 shows a side view of drill bit according to a first exemplary embodiment of the invention;
Fig. 6 shows a perspective view of the drill bit of Fig. 5;
Fig. 7 shows a side view of a drill bit according to another embodiment of the invention;
Fig. 8 shows a side view of a drill bit according to another embodiment of the invention;
Fig. 9 shows a side view of a drill bit according to another embodiment of the invention;
Fig. 10 shows a side view of a drill bit according to another embodiment of the invention;
Fig. 11 shows a side view of a chisel according to a first exemplary embodiment of the invention;
Fig. 12 shows a partial cross-sectional view of the chisel of Fig. 11;
Fig. 13 shows a perspective view of a chisel blade according to another embodiment of the invention;
Fig. 14 shows a side view of an osteotomy chisel according to another embodiment of the invention;
Fig. 15 shows a zoomed view of the osteotomy chisel of Fig. 14;
Fig. 16 shows a chart of conventional material compositions tested via a Schaeffler diagram for standard analysis;
Fig. 17 shows the Schaeffler diagram for the material of Fig. 16;
Fig. 18 shows a chart of conventional material compositions tested via a WRC-1992 diagram for standard analysis;
Fig. 19 shows the WRC-1992 diagram for the material of Fig. 18;
Fig. 20 shows a chart of an exemplary material composition according to the invention tested via a Schaeffler diagram for standard analysis;
Fig. 21 shows the Schaeffler diagram for the material of Fig. 20;
Fig. 22 shows a chart of the exemplary material composition according to the invention tested via a WRC-1992 diagram for standard analysis; and
Fig. 23 shows the WRC-1992 diagram for the material of Fig. 22;

### Detailed Description

The present invention may be further understood with reference to the following description and the appended drawings, wherein like elements are referred to with the same reference numerals. The present invention relates to device used to prepare a fractured or otherwise damaged bone to receive a bone fixation apparatus (e.g., a bone plate, bone screw, intramedullary nail, etc.). In particular, the present invention is directed to a bone cutting device (e.g., a reamer, chisel blade, drill, surgical needle, etc.) formed of implant-grade material and having a carburized or nitrided outer surface configured to increase a surface hardness thereof to a desired level. Implant-grade materials are those which are suitable for permanent implantation in the body - i.e., materials which will not have adverse health effects if left within the body for extended periods of time. The carburized or nitrided outer surface is selected to have a hardness greater than that of a bone being treated. In contrast to bone cutting devices which are formed of non-surface treated implant-grade material and often buckle or break when subjected to drilling, chiseling or reaming forces, exemplary bone cutting devices according to the invention are able to withstand increased levels of force without buckling or otherwise deforming. The exemplary bone cutting device according to the invention is formed with a carburized or nitrided outer surface which prevents the burring of threads or the dulling of sharpened surfaces during insertion into the bone, permitting the continued use of the same bone cutting device without sharpening or replacement. Furthermore, the exemplary implant-grade material of the invention provided a tactile feedback to prevent or inhibit breakage thereof. Specifically, the material of formed such that, when an excessive force is applied thereof, the device will undergo a degree of bending instead of shattering. Thus, a surgeon or other user may react to the bending and eliminate/ reduce the force being applied thereto to prevent breakage. The exemplary carburized or nitrided implant-grade material according to the invention offers the additional advantage of Furthermore, the implant-grade material of the present invention is selected so that even if a small fragment thereof were to inadvertently enter the body, removal would not be necessary, as will be described in greater detail hereinafter. In cases where the device does fracture, the exemplary material treatment according to the invention causes edges of fractured portion to be smoother and more rounded as comparted to non-treated materials reducing trauma to tissue. The exemplary bone fixation devices according to the invention provide an increased overall strength when compared to non-surface treated bone fixation devices formed out of implant-grade material, including increased yield strength, ultimate tensile strength and fatigue strength, as those skilled in the art will understand. It should be noted that the terms "proximal" and "distal" as used herein, are intended to refer to a direction toward (proximal) and away from (distal) a user of the device.

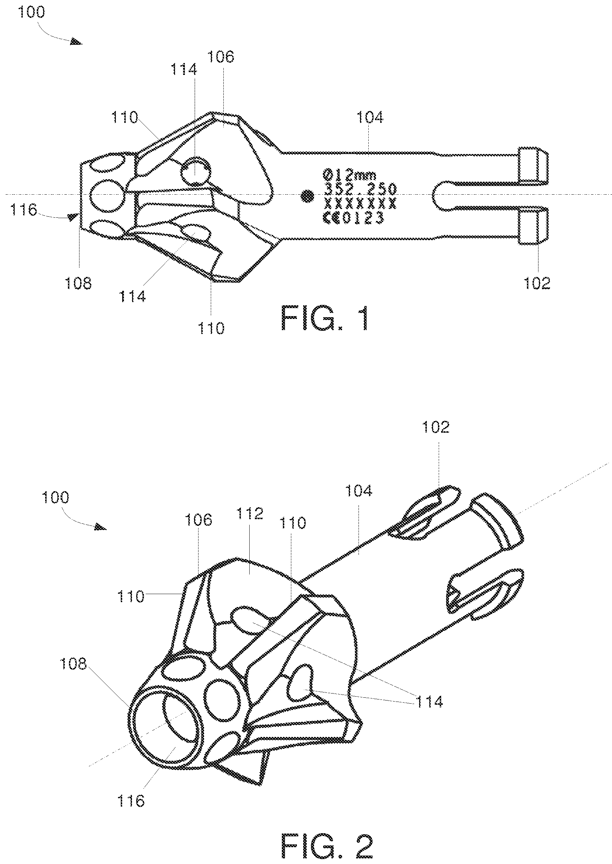

As shown in Figs. 1 - 2, a reamer head 100 according to a first exemplary embodiment of the invention extends from a proximal end 102 attachable to a reamer rod (not shown) and along an elongated shaft 104 to a head 106 adjacent a distal end 108. In an exemplary embodiment, an outer surface of the head 106 includes a plurality of sharp edges 110 for efficient cutting and deep flutes 112 to minimize clogging during a reaming procedure. The head 106 also comprises a plurality of openings 114 distributed thereabout to permit a flow of irrigation fluid out of the reamer head 100 to cool the head 100 and emulsify bone marrow for easy evacuation, as those skilled in the art will understand. A central longitudinal channel 116 extending through the reamer head 100 permits the flow of bone marrow and emulsified bone therethrough and out of the body. In an exemplary embodiment, the reamer head 100 is a 12 mm. head having five flutes 112. It is noted, however, that any diameter and number of flutes 112 may be provided without deviating from the scope of the invention.

The reamer head 100 is formed of an implant grade material including, but not limited to, implant quality austenitic stainless steel (e.g.,316L, 22-13-5, Biodur 108), cobalt alloys such as CCM (Co-28Cr-6Mo Alloy), MP35N, L605, ASTM-F-1058 and Elgiloy and Titanium and its alloys such as Ti-6Al-4V, Ti-6AI-7Nb and Ti-15Mo. The selected material is preferably not magnetic so that, if fragmented and left within the body, the patient may undergo magnetic resonance imaging ("MRI") without suffering adverse effects, as those skilled in the art will understand and at the discretion of a surgeon or other user. Furthermore, the carburized/nitrided treatment of the selected material results in fragmented portions that do not contain sharp edges, preventing trauma to surrounding tissue. While the selected material of the reamer head 100 is substantially soft when compared to conventional devices, the addition of a carburized or nitrided outer surface increases a rigidity thereof to a level greater than that of a bone with which it is to be employed and substantially greater than conventional reamer heads. Specifically, the reamer head 100 may have a surface hardness of approximately 68 HRC or more, as those skilled in the art will understand. In an exemplary embodiment, the hardness of the reamer head 100 may be approximately 67 - 74 HRC and, more particularly, 67.5 - 70.3 HRC. As those skilled in the art will understand, this configuration prevents dulling of the sharp edges 110 after prolonged use while also easing insertion of the reamer head 100 into the bone in accordance with an exemplary reaming procedure. During operation, the carburized or nitrided outer surface of the reamer head 100 aids in cutting through bone and/or metal without seizing or losing sharpness. The exemplary carburized or nitrided outer surface of the reamer head 100 permits repeated use in multiple bone fixation procedures. In contrast, conventional bone cutting devices must be replaced after one or a limited number of uses. Furthermore, the carburized or nitrided material of the present invention provides an increased rigidity to the bone cutting device without having to enlarge the device or otherwise change a geometry thereof.

The exemplary material according to the invention is treated using low-temperature carburization, which, in contrast with other treatment methods minimizes the formation of carbides. U.S. Patent No. 6,464,448 entitled "Low Temperature Case Hardening Process-", describes low temperature carburization of a ferrous based material for industrial parts and assemblies. However, these processes have not previously been applied to implant grade medical devices or implants. The present application applies low-temperature carburization of steel or other materials to provide a corrosion resistant material sufficient for use in surgical instruments. That is, the exemplary system and method according to the invention adapts a novel technique of carburizing/nitriding an implant-grade, ferrite free material to form devices having increased corrosion resistance as compared to other materials known in the art in which corrosion may be caused, for example, in part by the binding of chromium to carbide instead of being available to form an oxide. Higher levels of molybdenum in the material according to the invention further increase the corrosion-resistance thereof. As those skilled in the art will understand, a combination of annealing and cold-working may be used to form any of the devices described herein. The resultant material includes a diffusion zone in which carbon has supersaturated the matrix in the form of an interstitial carbon. The effect of this supersaturation is improved hardness, wear resistance and corrosion resistance. The exemplary material of the invention is described in greater detail below.

As those skilled in the art will understand, there are three main cubic forms of iron: austenite (FCC), Martensite (BCT) and ferrite (BCC). Both Martensite and ferrite are magnetic, while austenite is not. Thus, conventional implant quality 316L stainless steel is intentionally balanced to be fully austenitic even in the as-cast condition to minimize or eliminate the interaction of the medical device with MRI magnetic fields. This is done by balancing ferrite stabilizing elements with austenite stabilizing elements in such a way as to ensure that the as-cast balance is in the austenite region. It is well known that certain elements stabilize either austenite or ferrite. Since many of the ferrite stabilizing elements, such as molybdenum and chromium, also promote corrosion resistance, they must be balanced by increasing the austenite forming elements or the alloy will contain ferrite along with the austenite. Specification ranges may seem overly broad, however, when one balances the need to create certain phase balances with the need to maximize corrosion resistance while minimizing cost, it becomes evident that actual chemistries will vary in a much smaller range than the specifications imply. In conventional industrial versions of 316L, a certain amount of ferrite is purposely present in the alloy to improve welding characteristics of the alloy as ferrite is known to reduce hot cracking in welds. For the steelmaker, this provides a similar reduction of hot cracking during melting and casting, especially during continuous casting. The typical commercial 316L material is an alloy that contains a majority austenite with a small percentage of ferrite. This is true in the as-cast condition and also as finished wrought products such as bar, wire, sheet and plate.

On the other hand, implant quality 316L is chemically balanced so that no ferrite is present in the alloy. Although the chemical ranges given in specifications such as ASTM F 138 are capable of producing ferrite, the specifications require that the end product contain no ferrite. To accomplish this, producers balance the actual chemistry into the 100% austenite region. There are many methods for predicting the austenite-ferrite balance in stainless steels. Two of the most common are the Schaeffler and the WRC-1992 diagrams. In each of these techniques, a correlation has been made between the chrome equivalent, the nickel equivalent and the phase balance. The chrome and nickel equivalents relate the total amount of ferrite or austenite forming elements present to their stabilizing effect in relation to the base elements of chrome and nickel. Carburization is a diffusion controlled process wherein only a small region near an exterior surface layer of a device on the order of 20 µm -35µm thick is carburized. If a ferrite grain remains present in this region, it will not be carburized forming an uncarburized area that will not be as corrosion resistant as the carburized layer. Corrosion tunneling effects can occur in these areas allowing corrosion to penetrate to the core of the item potentially resulting in catastrophic failure.

The exemplary material according to the invention utilizes implant quality 316L for carburizing as it does not contain any ferrite, thus mitigating the risk of the presence of ferrite particles disrupting the carburized layer. To show the propensity for formation of ferrite, the following were compared: (1) implant quality 316L received at Synthes that meets the requirements of ASTM F138, ASTM F 139 and ISO 5832-1. Sample size - 1366 samples and (2) industrial quality 316L produced by a supplier to the requirements of ASTM A 276. - Samples size - 3,556 samples. The average chemistry of each was plotted to determine the ferrite content using the Schaeffler and the WRC-1992 methods, as shown in the following tables:

| **Industrial Quality 316L** | | | |
|---|---|---|---|
| | **N** | **Average** | **A 276 Limits** |
| **C** | 3557 | 0.023467 | 0.08 max |
| **Mn** | 3558 | 1.460599 | 2.00 max |
| **P** | 3558 | 0.027386 | 0.045 max |
| **S** | 3557 | 0.022573 | 0.03 max |
| **Si** | 3558 | 0.577813 | 1.00 max |
| **Cr** | 3558 | 16.90085 | 16.0-18.0 |
| **Ni** | 3558 | 10.37691 | 10.0-14.0 |
| **Mo** | 3556 | 2.077068 | 2.00-3.00 |

| **Implant Quality 316L** | | | |
|---|---|---|---|
| | **N** | **Average** | **ASTM F 138 Limits** |
| **C** | 1366 | 0.017471 | 0.030 max |
| **Mn** | 1366 | 1.677483 | 2.00 max |
| **P** | 1366 | 0.017485 | 0.025 max |
| **S** | 1366 | 0.001069 | 0.010 max |
| **Si** | 1366 | 0.420132 | 0.75 max |
| **Cr** | 1366 | 17.46971 | 17.00-19.00 |
| **Ni** | 1366 | 14.46273 | 13.00-15.00 |
| **Mo** | 1366 | 2.771717 | 2.25-3.00 |
| **N** | 1366 | 0.073903 | 0.10 max |
| **Cu** | 1366 | 0.101012 | 0.50 max |
| **Cr + 3.3Mo** | 1366 | 26.6164 | 26.0 min |

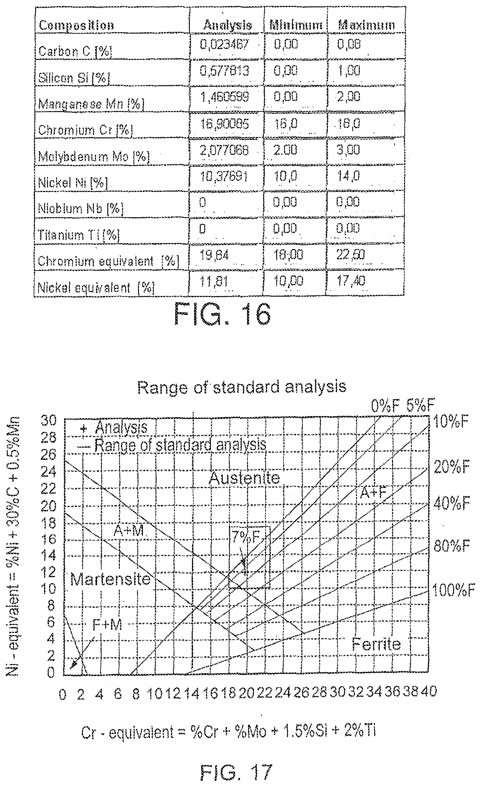

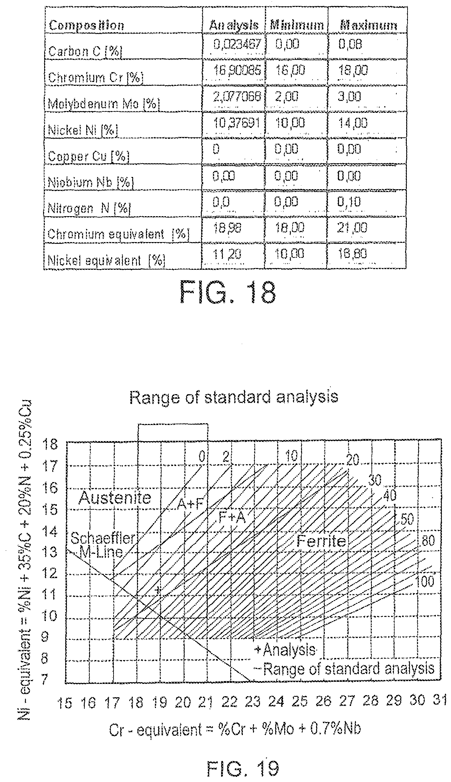

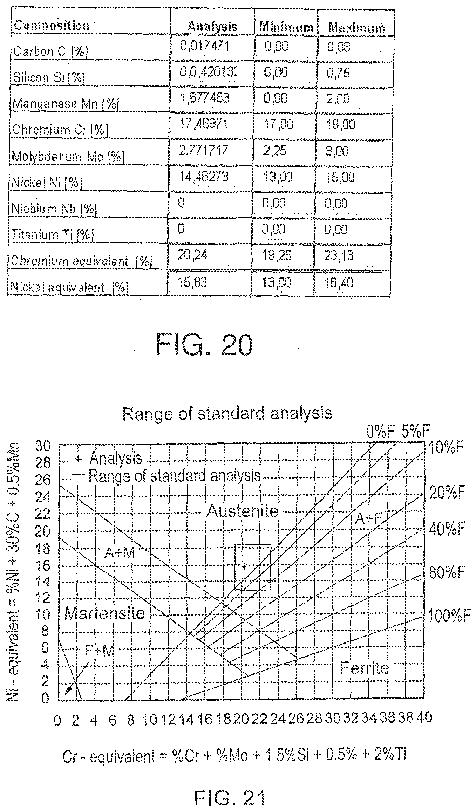

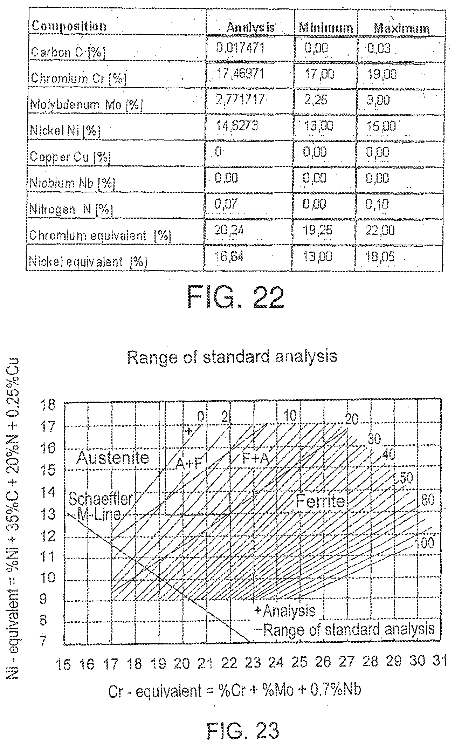

As depicted in Figs. 16 - 23, the comparison of the above disclosed materials show that the implant quality 316L is balance into the 100% austenite region, while the industrial quality 316L is balanced at approximately 7-8% ferrite. Each of the techniques also shows the possible % ferrite band based on the specification ranges. Specifically, Figure 17 provides information on the welding properties of various types of conventional industrial strength microstructures of Figure 16 as a function of the alloying elements they contain. The chart of Figure 17 corresponds to a Schaeffler diagram for a range of standard analysis for the following material compositions. Figure 19 provides information on welding properties of the various types of conventional industrial strength microstructures of Figure 18 as a function of the alloying elements they contain. The chart of Figure 19 corresponds to a WRC-1992 diagram for a range of standard analysis for the following material compositions. Figures 20 - 23 provide the same data for the exemplary implant grade material according to the invention, wherein Figure 21 corresponds to a Schaeffler diagram of the data of Figure 20 and Figure 23 corresponds to a WRC-1992 of the data of Figure 22. In light of the above, it is evident that the exemplary material according to the invention provides an implant grade material that is balanced in a 100% austenite region, eliminating the ferrite commonly produced in conventional materials.

Although the exemplary construction depicted herein is directed to a reamer head 100, the inventive concept may be employed with any other bone cutting device without deviating from the scope of the invention. Such bone cutting devices include, but are not limited to, saw blades, screwdrivers, osteotomes, chisels, drill bits, suture needles, syringe needles and other cutting or puncturing devices.

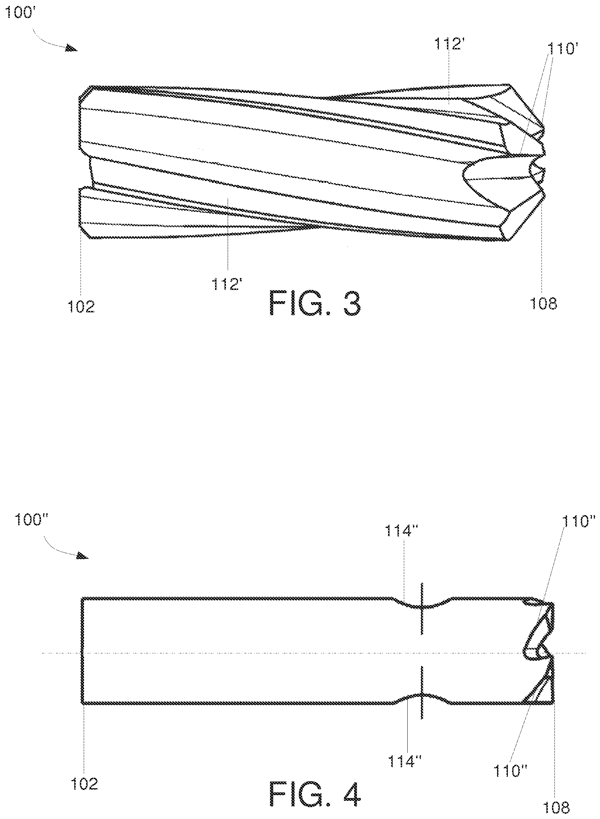

Fig. 3 depicts a reamer head 100' according to an alternate embodiment of the invention. The reamer head 100' is formed substantially similar to the reamer head 100 except as noted below. An outer surface of the reamer head 100' comprises a plurality of flutes 112' extending therealong from the proximal end 102 to the distal end 108. Sharpened cutting edges 110' are provided adjacent the distal end 108 to aid in cutting through tissue. The reamer head 100' is substantially cylindrical and is configured for attachment to a reamer rod known in the art, as those skilled in the art will understand. A material and overall rigidity of the reamer head 100' is substantially similar to that of reamer head 100, as described in greater detail earlier.

Fig. 4 depicts a reamer head 100" according to yet another alternate embodiment of the invention. The reamer head 100" is formed substantially similarly to the reamer heads 100, 100' except as noted below. An outer surface of the reamer head 100" is substantially smooth and comprises first and second openings 114" extending therethrough, opening axes of the first and second openings being aligned with one another and extending substantially transverse to a longitudinal axis of the reamer head 100". The reamer head 100" includes sharpened cutting edges 110" adjacent the distal end 108 to aid in cutting through tissue. A material and overall rigidity of the reamer head 100" is substantially similar to that of reamer head 100, as described in greater detail earlier. It is noted that any of the reamer heads 100, 100', 100" may be substantially hollow to permit retrieval of bone and/or bone screws, drill bits therethrough, as those skilled in the art will understand.

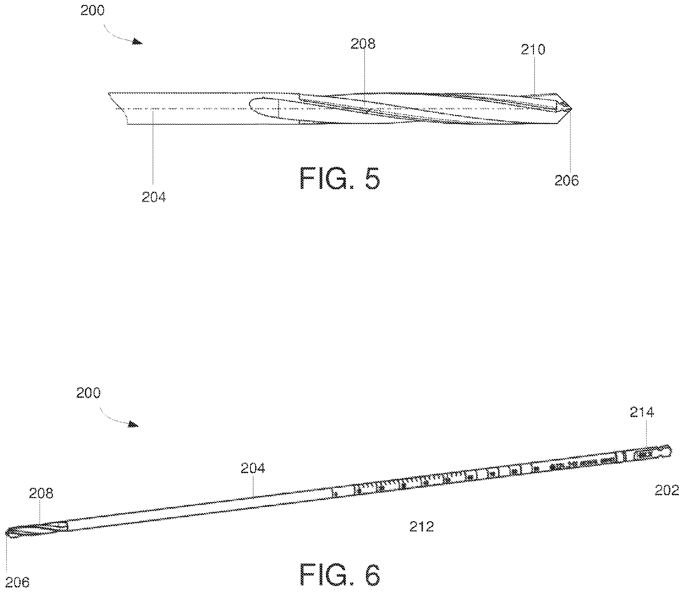

Figs. 5 - 6 depict an exemplary drill bit 200 according to another alternate embodiment of the invention. The drill bit 200 is formed of an implant grade material having a carburized or nitrided outer surface, as described in greater detail earlier with respect to the reamer head 100. The drill bit 200 extends from a proximal end 202 and along an elongated cylindrical shaft 204 to a distal end 206 having a plurality of flutes 208, each having a respective outer cutting surface 210. The drill bit 200 may include etching 212 along the shaft 204 to indicate a depth of insertion thereof, as those skilled in the art will understand. The proximal end 202 may include an attachment portion 214 permitting attachment thereof to a drilling mechanism known in the art. As with the reamer head 100, the drill bit 200 may be used in any plurality of procedures without losing sharpness or being damaged. In cases where the drill bit 200 does become damaged, the implant grade material permits that it is not necessary to remove any burred portions thereof from the body.

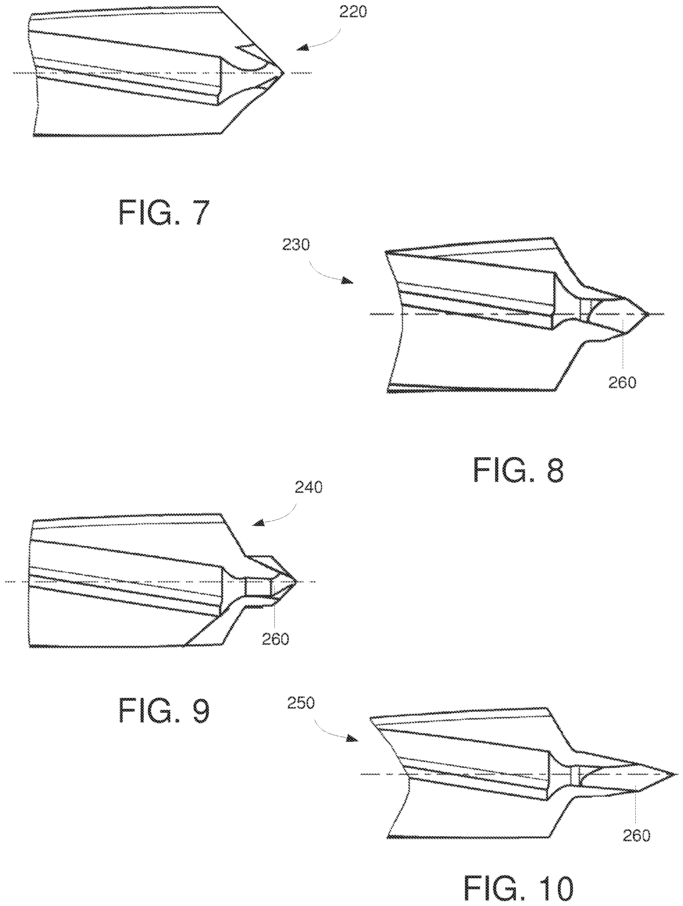

Although the drill bit 200 is shown with a particular geometry, it is noted that the inventive concept disclosed herein may be applied to any drill bit having any size or shape without deviating from the scope of the invention. For example, Figs. 7 - 10 disclose drill bits 220, 230, 240, 250 having varying geometries, all of which can be formed of the exemplary carburized or nitrided implant grade material of the invention. In some embodiment, the drill bits 230, 240, 250 may include projections 260 extending distally from the drill bit, the projections 260 having a diameter reduced relative to a diameter of a proximal portion of the drill bit 230, 240, 250. The flutes 208 may extend over the projections 260. It is noted that these examples are exemplary only and are not intended to limit the scope of the invention.

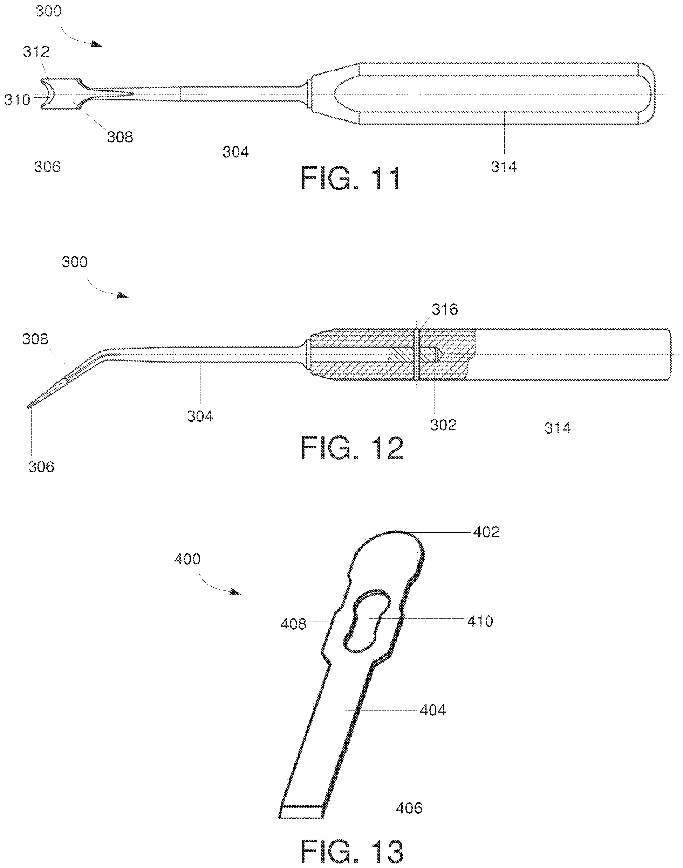

Figs. 11 - 12 depict a chisel 300 according to another embodiment of the invention, the chisel 300 extending from a proximal end 302 and along an elongated shaft 304 to a distal end 306 having an increased width paddle portion 308. A distal end of the paddle portion 308 may include a recess portion 310 with a sharpened cutting surface 312 configured to chisel bone. As shown in Fig. 12, the paddle portion 308 may be angled relative to the shaft 304 by approximately 38E, although any other angle may be used without deviating from the scope of the invention. A proximal length of the chisel 300 may be inserted into a corresponding opening formed through a handle 314 and secured thereto using a known attachments means (e.g., friction fit, adhesive, etc.), as those skilled in the art will understand. A screw or pin 316 may extend transversely through the proximal portion of the chisel 300 and the handle 314 to further secure the chisel therein. The exemplary chisel 300 is formed of a carburized or nitrided implant grade material, as described in greater detail earlier. In an operative configuration, the sharpened cutting surface 312 may be used to cut into bone without becoming dull or fracturing.

Fig. 13 depicts a chisel blade 400 according to an alternate embodiment of the invention. The chisel blade 400 extends from a proximal end 402 and along an elongated body 404 to a sharpened distal end 406. An increased width attachment portion 408 may include an opening 410 extending therethrough to engage an abutment portion (not shown) of a handle (not shown) to aid in manipulation thereof, as those skilled in the art will understand. The chisel blade 400 is formed of a carburized or nitrided implant grade material, as described in greater detail earlier. As those skilled in the art will understand, the depicted geometry of the chisel blade is exemplary only and any variations in geometry may be applied without deviating from the scope of the invention to, for example, suit the requirements of a particular procedure.

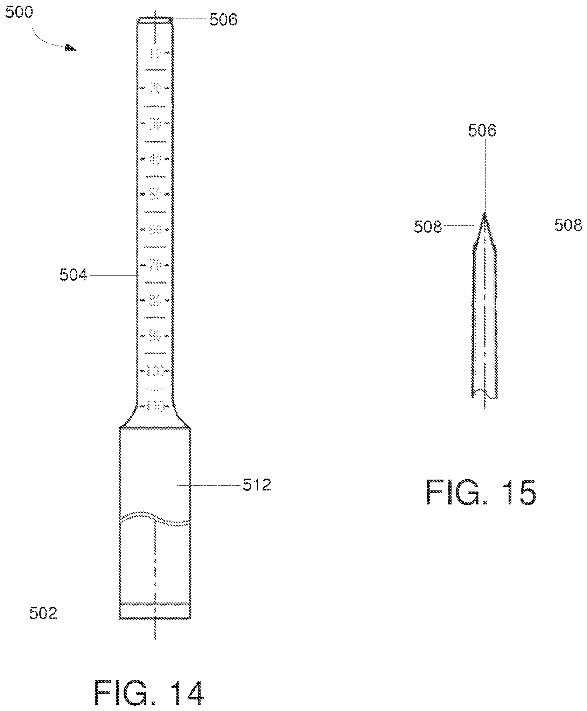

Figs. 14 - 15 depict an osteotomy chisel 500 according to yet another embodiment of the invention. The chisel 500 extends from a proximal end 502 and along an elongated body 504 to a sharpened distal tip 506 defined by first and second recessed portions 508 . The body 504 includes a plurality of markings 510 providing a visual guide of a depth of insertion of the chisel 500 into the bone, as those skilled in the art will understand. The proximal end of the chisel 500 includes an increased width portion 512 which, in an operative configuration, may be impacted via a hammer or other impacting tool to chisel into the bone, as those skilled in the art will understand. The chisel 500 is formed of a carburized or nitrided implant grade material, as described in greater detail earlier. As those skilled in the art will understand, the depicted geometry of the chisel is exemplary only and any variations in geometry may be applied without deviating from the scope of the invention to, for example, suit the requirements of a particular procedure.

It will be apparent to those skilled in the art that various modifications and variations can be made in the structure and the methodology of the present invention without departing from the scope of the invention as defined by the appended claims. Thus, it is intended that the present invention cover the modifications and variations of this invention provided that they come within the scope of the appended claims.

## Claims

1. A bone cutting device (100; 200; 300; 400; 500) extending from a proximal end (102; 202; 302; 402; 502) to a distal end (108; 206; 306; 406; 506) and having a sharpened cutting surface (110; 210; 312; 406; 506) adjacent the distal end (108; 206; 306; 406; 506), the cutting surface (110; 210; 312; 406; 506) being dimensioned to permit cutting of bone, the bone cutting device (100-500) being formed of a ferrite free implant grade material, **characterized in that** the ferrite free implant grade material is one of implant quality austenitic stainless steel and cobalt alloys such as CCM (Co-28Cr-6Mo Alloy), MP35N, L605, ASTM-F-1058 and Elgiloy, wherein an outer surface of the bone cutting device (100 - 500) is one of carburized and nitrided.

2. The device of claim 1, wherein the bone cutting device (100 - 500) is formed via a combination of annealing and cold-working.

3. The device of claim 1 or 2, wherein the hardness of the bone cutting device (100 - 500) is in the range of 67 - 74 HRC.

4. The device of any one of claims 1 to 3, wherein the bone cutting device (100) is a reamer head having plurality of flutes (112) distributed along an outer surface thereof.

5. The device of claim 4, wherein each flute (112) includes a corresponding cutting surface (110) on an outer portion thereof or the cutting surface (110') is provided on a distal end of each of the flutes (112').

6. The device of claim 4 or 5, further comprising an irrigation opening (114) extending through the device (100) to direct a flow of irrigation fluid therethrough and onto a target cutting area.

7. The device of any one of claims 1 to 6, further comprising a channel (116) extending through the device (100).

8. The device of any one of claims 1 to 4, wherein the bone cutting device (200) is an elongated drill bit.

9. The device of claim 8, wherein a proximal portion of the drill bit (200) includes marking (212) indicating a depth of insertion thereof into bone.

10. The device of claim 8 or 9, wherein the drill bit (230; 240; 250) is substantially cylindrical.

11. The device of claim 10, wherein a distal tip of the drill tip (230; 240; 250) includes a projection (260) extending distally thereoutof, a diameter of the projection (260) being smaller than a diameter of a proximal portion thereof.

12. The device of any one of claims 1 to 4, wherein the bone cutting (300; 400; 500) device is a chisel (300; 400; 500), preferably an osteotomy chisel.

13. The device of claim 12, wherein the cutting surface (312; 406; 506) is formed on a distal edge (306; 406; 506) of the chisel (300; 400; 500), wherein preferably the cutting surface (310) is substantially curved.

14. The device of claim 12 or 13, further comprising an increased diameter paddle portion (308) adjacent the distal end (306), the paddle portion (308) being angled with respect to a shaft portion (304) of the chisel (300).

15. The device of claim 13, wherein the distal cutting surface (506) is defined by first and second opposing angled walls (508) converging at the distal end (506).

## Patentansprüche

1. Knochenschneidvorrichtung (100; 200; 300; 400; 500), die sich von einem proximalen Ende (102; 202; 302; 402; 502) zu einem distalen Ende (108; 206; 306; 406; 506) erstreckt und eine geschärfte Schneidoberfläche (110; 210; 312; 406; 506) benachbart zum distalen Ende (108; 206; 306; 406; 506) aufweist, wobei die Schneidoberfläche (110; 210; 312; 406; 506) dimensioniert ist, ein Schneiden von Knochen zu erlauben, wobei die Knochenschneidvorrichtung (100-500) aus einem ferritfreien Material in Implantatqualität besteht,
**dadurch gekennzeichnet, dass** das ferritfreie Material in Implantatqualität entweder austenitischer Edelstahl in Implantatqualität oder eine Kobaltlegierung, wie CCM (Co-28Cr-6Mo Legierung), MP35N, L605, ASTM-F-1058 und Elgiloy, ist, wobei eine äußere Oberfläche der Knochenschneidvorrichtung (100 - 500) entweder karburiert oder nitriert ist.

2. Vorrichtung nach Anspruch 1, wobei die Knochenschneidvorrichtung (100 - 500) durch eine Kombination aus Glühen und Kaltumformen gebildet ist.

3. Vorrichtung nach Anspruch 1 oder 2, wobei die Härte der Knochenschneidvorrichtung (100 - 500) im Bereich von 67 - 74 HRC ist.

4. Vorrichtung nach einem der Ansprüche 1 bis 3, wobei die Knochenschneidvorrichtung (100) ein Kopf einer Reibahle ist, der eine Mehrzahl von Nuten (112) aufweist, die entlang einer äußeren Oberfläche davon verteilt sind.

5. Vorrichtung nach Anspruch 4, wobei jede Nut (112) eine entsprechende Schneidoberfläche (110) an einem äußeren Teil davon aufweist oder die Schneidoberfläche (110') an einem distalen Ende jeder der Nuten (112') vorgesehen ist.

6. Vorrichtung nach Anspruch 4 oder 5, des Weiteren umfassend eine Spülöffnung (114), die sich durch die Vorrichtung (100) hindurch erstreckt, um einen Strom einer Spülflüssigkeit dort hindurch und auf einen Zielschneidbereich zu leiten.

7. Vorrichtung nach einem der Ansprüche 1 bis 6, des Weiteren umfassend einen Kanal (116), der sich durch die Vorrichtung (100) hindurch erstreckt.

8. Vorrichtung nach einem der Ansprüche 1 bis 4, wobei die Knochenschneidvorrichtung (200) ein länglicher Bohreinsatz ist.

9. Vorrichtung nach Anspruch 8, wobei ein proximaler Teil des Bohreinsatzes (200) eine Markierung (212) enthält, die eine Einführtiefe davon in Knochen angibt.

10. Vorrichtung nach Anspruch 8 oder 9, wobei der Bohreinsatz (230; 240; 250) im Wesentlichen zylindrisch ist.

11. Vorrichtung nach Anspruch 10, wobei eine distale Spitze des Bohreinsatzes (230; 240; 250) einen Vorsprung (260) enthält, der sich distal davon weg erstreckt, wobei ein Durchmesser des Vorsprungs (260) kleiner ist als ein Durchmesser eines proximalen Teils davon.

12. Vorrichtung nach einem der Ansprüche 1 bis 4, wobei die Knochenschneidvorrichtung (300; 400; 500) ein Meißel (300; 400; 500) ist, vorzugsweise ein Osteotomie-Meißel.

13. Vorrichtung nach Anspruch 12, wobei die Schneidoberfläche (312; 406; 506) an einem distalen Rand (306; 406; 506) des Meißels (300; 400; 500) gebildet ist, wobei die Schneidoberfläche (310) vorzugsweise im Wesentlichen gekrümmt ist.

14. Vorrichtung nach Anspruch 12 oder 13, des Weiteren umfassend einen Paddelteil (308) mit vergrößertem Durchmesser benachbart zum distalen Ende (306), wobei der Paddelteil (308) bezüglich eines Schaftteils (304) des Meißels (300) abgewinkelt ist.

15. Vorrichtung nach Anspruch 13, wobei die distale Schneidoberfläche (506) durch erste und zweite gegenüberliegende abgewinkelte Wände (508) definiert ist, die am distalen Ende (506) konvergieren.

## Revendications

1. Dispositif de coupe d'os (100; 200; 300; 400; 500) s'étendant d'une extrémité proximale (102; 202; 302; 402; 502) à une extrémité distale (108; 206; 306; 406;506) et présentant une surface coupante aiguisée (110; 210; 312; 406; 506) adjacente à l'extrémité distale (108; 206; 306; 406;506), la surface coupante aiguisée (110; 210; 312; 406; 506) étant dimensionnée pour permettre la coupe d'os, le dispositif de coupe d'os (100-500) étant formé d'un matériau de qualité pour implant exempt de ferrite, **caractérisé en ce que** le matériau de qualité pour implant exempt de ferrite est un élément parmi des alliages austénitiques d'acier inoxydable et de cobalt de qualité pour implant tels que CCM (alliage Co-28Cr-6Mo), MP35N, L605, ASTM-F-1058 et Elgiloy, dans lequel une surface extérieure du dispositif de coupe d'os (100-500) est soit carburée, soit nitrurée.

2. Dispositif selon la revendication 1, dans lequel le dispositif de coupe d'os (100-500) est formé par une combinaison de recuit et de formage à froid.

3. Dispositif selon la revendication 1 ou 2, dans lequel la dureté du dispositif de coupe d'os (100-500) est comprise dans l'intervalle de 67-74 HRC.

4. Dispositif selon l'une quelconque des revendications 1 à 3, dans lequel le dispositif de coupe d'os (100) est une tête d'alésoir présentant une pluralité de goujures (112) réparties le long de la surface extérieure de celle-ci.

5. Dispositif selon la revendication 4, dans lequel chaque goujure (112) inclut une surface coupante correspondante (110) sur une portion extérieure, ou la surface coupante (110') est prévue sur une extrémité distale de chacune des goujures (112').

6. Dispositif selon la revendication 4 ou 5, comprenant en outre une ouverture d'irrigation (114) s'étendant à travers le dispositif (100) pour diriger un flux de fluide d'irrigation à travers elle et vers une zone de coupe cible.

7. Dispositif selon l'une quelconque des revendications 1 à 6, comprenant en outre un canal (116) s'étendant à travers le dispositif (100).

8. Dispositif selon l'une quelconque des revendications 1 à 4, dans lequel le dispositif de coupe d'os (200) est un foret oblong.

9. Dispositif selon la revendication 8, dans lequel une portion proximale du foret (200) inclut un repère (212) indiquant une profondeur d'introduction de celui-ci dans l'os.

10. Dispositif selon la revendication 8 ou 9, dans lequel le foret (230; 240; 250) est en grande partie cylindrique.

11. Dispositif selon la revendication 10, dans lequel un bout distal du foret (230; 240; 250) inclut une saillie (260) s'étendant de manière distale hors de celui-ci, un diamètre de la saillie (260) étant inférieur à un diamètre d'une portion proximale de celui-ci.

12. Dispositif selon l'une quelconque des revendications 1 à 4, dans lequel le dispositif de coupe d'os (300; 400; 500) est un ciseau (300; 400; 500), de préférence un ciseau pour ostéotomie.

13. Dispositif selon la revendication 12, dans lequel la surface coupante (312; 406; 506) est formée sur un bord distal (306; 406, 506) du ciseau (300; 400; 500), dans lequel de préférence, la surface coupante (310) est en grande partie incurvée.

14. Dispositif selon la revendication 12 ou 13, comprenant en outre une portion de spatule de diamètre augmenté (308) adjacente à l'extrémité distale (306), la portion de spatule (308) formant un angle par rapport à une partie de manche (304) du ciseau (300).

15. Dispositif selon la revendication 13, dans lequel la surface coupante distale (506) est définie par des première et seconde parois présentant un angle et opposées (508) convergeant sur l'extrémité distale (506).
